# EUROPÄISCHE PATENTSCHRIFT

(11) **EP 3 793 526 B1**
(45) Veröffentlichungstag und Bekanntmachung des Hinweises auf die Patenterteilung: **14.02.2024**
(21) Anmeldenummer: 19723427.1
(22) Anmeldetag: 15.05.2019
(51) Int. Cl.: A61K 9/16, A61K 9/20, A61K 9/28, C11C 3/00

(54) **POLY(METH)ACRYLSÄURE-POPCORNPOLYMERISATE ALS SPRENGMITTEL FÜR TABLETTEN**
POLY(METH)ACRYLIC ACID POPCORN POLYMERIZATES AS DISINTEGRANTS FOR TABLETS
POLYMÉRISATS DE TYPE POPCORN D'ACIDE POLY(MÉTH)ACRYLIQUE COMME DÉSINTÉGRANTS POUR COMPRIMÉS

(30) Priorität: 16.05.2018 EP 18172745
(43) Veröffentlichungstag der Anmeldung: 24.03.2021
(73) Patentinhaber: BASF SE, 67056 Ludwigshafen am Rhein (DE)
(72) Erfinder: SMIT, Theo, 67056 Ludwigshafen (DE); KARL, Matthias, 67056 Ludwigshafen (DE); GUTH, Felicitas, 67056 Ludwigshafen (DE); ANGEL, Maximilian, 95359 Kasendorf (DE); KOLTER, Karl, 67056 Ludwigshafen (DE); SCHMIDT, Frank, 67056 Ludwigshafen (DE); BLOCHBERGER-CLAUS, Maximilian, 67056 Ludwigshafen (DE)
(74) Vertreter: BASF IP Association
(86) Internationale Anmeldenummer: PCT/EP2019/062407
(87) Internationale Veröffentlichungsnummer: WO 2019/219717

(56) Entgegenhaltungen:
- EP-A1- 0 177 812
- EP-A1- 1 035 196
- EP-A1- 1 167 433
- EP-B1- 0 177 812
- EP-B1- 1 035 196
- DE-A1- 4 237 439
- DE-A1- 10 011 137

## Beschreibung

Die vorliegende Erfindung betrifft die Verwendung von wasserunlöslichen, nicht quellenden Poly(meth)acrylsäure-Popcornpolymerisaten als Sprengmittel für pharmazeutische Tabletten.

Acrylsäure-Popcornpolymere sind an sich bekannt, siehe J.W. Breitenbach et al., in "Die Makromolekukare Chemie", 175, 2597-2604 (1974) oder in J.W. Breitenbach, Makromolekulare Chemie, 177, 2787-2792 (1976), ohne dass eine Verwendung beschrieben wurde.

In der EP-A 177 812 wird die Herstellung von Popcorn-Polymerisaten auf Basis von Carbonsäuren und deren Derivaten, beispielsweise Acrylsäurederivaten, durch Polymerisation in einem Festbett unter Verwendung von aus N-Vinylpyrrolidon-2 erhaltenen Popcornpolymerisaten als Saatgut, beschrieben. Solche Polymerisate sollen sich als Adsorbentien oder als Formulierungshilfsmittel für Pflanzenschutzmittel eignen, wobei konkret die Adsorption von Tanninen beschrieben ist.

Die Verwendung von Sprengmitteln zur Verbesserung des Zerfalls und der Auflösungsgeschwindigkeit von Tabletten ist seit langem allgemein bekannt.

Ein bewährtes Sprengmittel ist dabei vernetztes Polyvinylpyrrolidon, auch als PVPP oder Crospovidon bezeichnet, wie beispielsweise in der US 6,677,417 eq. DE 10011137A1 beschrieben. Dabei handelt es sich um Popcorn-Polymerisate. Crospovidon zeigt eine starke Quellfähigkeit und hervorragende Sprengmitteleigenschaften, ist aber eher hochpreisig.

Starke Quellfähigkeit besitzt auch das in der EP-A 1167433 beschriebene unlöslich, vernetzte Polyacrylat. Kostengünstigere kommerziell verfügbare Sprengmittel sind beispielsweise Croscarmellose-Na, das Natriumsalz einer quervernetzten Carboxymethylstärke, oder Natrium-Stärkeglycolat, auch wenn deren Sprengwirkung geringer ist als die von Crospovidon.

Weiterhin ist die Verwendung von vernetzter Polyacrylsäure vom Typ eines Kationenaustauscherharzes als Sprengmittel bekannt. Solche Harze sind aus den Pharmakopöen auch als Polacrilin Potassium NF bekannt. Ein kommerziell verfügbares Produkt dieses Typs ist beispielsweise Amberlite TM IRP 88, Fa. Dow Chemicals, auf Basis von Methacrylsäure vernetzt mit Divinylbenzol. Die Sprengmittelwirkung wird dabei dem Quellverhalten bei Hydratation zugeschrieben. Allerdings weisen wässrige Suspensionen solcher Polacrilin-Harze relativ hohe pH-Werte auf, sodass sie für hydrolyseempfindliche Wirkstoffe wenig geeignet sind.

Zur Verwendung von Stärkederivaten oder Polacrilin-Harzen als Sprengmittel siehe auch K.A. Khan and C.T. Rhodes, J. of Pharmaceutical Sciences, Vol. 64, No. 3, 447-451 (1975).

Aus der EP-A 1035 196, der EP-A 1314776 und der EP-A 972825 sind vernetzte Polyacrylate und deren Verwendung als Sprengmittel für Pelletformulierungen bekannt, wobei die vernetzten Polyacrylate eine Gelbildungszeit von 30 Sekunden oder weniger aufweisen sollen. Allerdings sind solche rasch gelbildenden vernetzten Polyacrylate hinsichtlich der Zerfallszeit von Tabletten nachteilig.

Aus der DE 4237439A1 sind unlösliche, nur wenig quellbare Polymerisate mit Aminogruppen bekannt. Diese Polymerisate werden hergestellt, indem man offenkettige N-Vinylcarbonsäureamide und gegebenenfalls andere, damit copolymerisierbare Monomere mit 0,1 bis 10 Gew.-%, bezogen auf die bei der Polymerisation eingesetzten Monomeren, einer mindestens zwei ethylenisch ungesättigte Doppelbindungen aufweisenden Verbindung als Vernetzer unter Ausschluss von Sauerstoff und Polymerisationsinitiatoren zu Popcornpolymerisaten polymerisiert und aus mindestens 2% der einpolymerisierten N-Vinylcarbonsäureamide die Formyl- bzw. Acylgruppe unter Bildung von 65 Aminogruppen enthaltenden Popcornpolymeren durch Einwirkung von Enzymen, Basen oder Säuren abspaltet. Die Popcornpolymerisate werden als Ionenaustauscher oder als Adsorberharz für Metallionen verwendet.

Aufgabe der vorliegenden Erfindung war es, ein Tablettensprengmittel zu finden, welches eine mit Crospovidone vergleichbare gute Sprengwirkung zeigt, dabei aber kostengünstiger verfügbar ist, eine bessere Sprengwirkung hat als vergleichbar kostengünstige Produkte wie zum Beispiel die Stärkederivate und weiterhin auch für hydrolyseempfindliche Arzneistoffe eingesetzt werden kann.

Demgemäß wurde die Verwendung von wasserunlöslichen, wenig quellenden Polyacrylaten als Sprengmittel für pharmazeutische Dosierungsformen, insbesondere Tabletten gefunden. "Polyacrylate" bezeichnet im Sinne der Erfindung Polymerisate auf Basis von Acrylsäure oder Methacrylsäure oder deren Gemischen.

Weiterhin können in die Polyacrylate auch bis zu 20 Gew.-% Strukturelemente von Comonomeren enthalten. Comonomere können ausgewählt sein aus der Gruppe bestehend aus Methacrylsäureestern wie Methylacrylat, Methylmethacrylat, Hydroxyethylmethacrylat, Hydroxyethylacrylat und Ethylacrylat, Butylacrylat, 2-Ethylhexylacrylat.

Besonders bevorzugt sind Polymerisate aus reiner Acrylsäure oder Methacrylsäure.

"Wasserunlöslich" bedeutet erfindungsgemäß, dass weniger als 1 % (m/m) des Polymers bei 20 °C und Normaldruck in Wasser löslich ist. "Wenig quellend" bedeutet erfindungsgemäß, dass die prozentuale Änderung der Teilchengröße der Polymere in Wasser im Vergleich zur Änderung der Teilchengröße in Hexadecan bei kleiner 50 % liegt. Die Messung der prozentualen Teilchengrößenveränderung kann nach ISO 13320:2009 durchgeführt werden, wie nachstehend näher ausgeführt.

Bei den erfindungsgemäß verwendeten Polyacrylaten handelt es sich um Popcorn-Polymerisate.

Die erfindungsgemäß verwendeten Polymerisate sind weiterhin langsam gelbildend. Langsam gelbildend bedeutet, dass die Gelbildungszeit mindestens mehr als 30 Sekunden beträgt. Die Gelbildungszeit kann wie aus dem Stand der Technik bekannt durchgeführt werden. Dabei wird die Zeit bestimmt, nach der die durch das Rühren verursachte Wirbelröhre in einer Testflüssigkeit nicht mehr sichtbar ist. Als Testflüssigkeit wird eine 0,9 Gew.-%ige Natriumchloridlösung verwendet.

Die Bestimmung der Gelbildungszeit kann wie in der EP-A 1035196, [0028], beschrieben erfolgen.

Eine wässrige Lösung von Natriumchlorid wird durch Lösen von Natriumchlorid mit analytischem Reinheitsgrad in demineralisiertem Wasser bis zu einer Konzentration von 0,9 Gew.-% des Salzes in der Endlösung zubereitet. Diese wässrige 0,9 Gew.-% NatriumchloridLösung ist eine Bezugstestflüssigkeit ("Testflüssigkeit"), die häufig in der Charakterisierung von wasserabsorbierenden Polymeren eingesetzt wird. In einem Raum mit kontrollierter Temperatur (20°C) wird das wasserabsorbierende, quervemetzte Polyacrylatpolymer (3 g) in ein Becherglas (100 ml) mit einem Innendurchmesser von 55 mm eingeführt. Das Becherglas wird über einem Elektromagnetrührer platziert und ein Magnetrührstab (45 mm x 8 mm) wird für das Rühren hineingegeben. Die Geschwindigkeit des Rührens wird auf 600 +/- 20 rpm eingestellt, und das Becherglas wird rasch mit der Testflüssigkeit (50 g) gefüllt. Sobald die Zugabe der Testflüssigkeit abgeschlossen ist, wird dieser Zeitpunkt als Zeitpunkt Null festgehalten, also dem Zeitpunkt, von dem an die Zeit gemessen wird. Die Zeitmessungen werden gestoppt, wenn das Rühren (stirring vortex) durch die Bildung eines Gels durch das wasserabsorbierende Polymer nicht mehr sichtbar ist, das heißt, wenn es keine Wirbelröhre mehr gibt. Bei schnell gelbildenden Polymeren kann es erforderlich sein, die Reihenfolge der Zugabe von Polymer und Testflüssigkeit in das Becherglas umzukehren.

Die erfindungsgemäß verwendeten Poly(meth)acrylate werden durch proliferierende Polymerisation erhalten. Eine solche Polymerisation wird auch als Popcorn-Polymerisation bezeichnet. Die Popcorn-Polymerisation führt zu einer starken physikalischen Vernetzung durch Verschlaufung der Polymerketten. Die entstehenden Polymerpartikel haben eine blumenkohlartige Struktur.

Die Polymerisation wird vorzugsweise in Abwesenheit von Sauerstoff durchgeführt. "In Abwesenheit von Sauerstoff" bedeutet, dass die Sauerstoffkonzentration in der Gasphase in der Polymerisationsvorrichtung so niedrig ist, dass spontane Radikalbildung erfolgen kann, ohne dass die spontan entstandenen Radikale gleich mit Sauerstoff reagieren. Die Abwesenheit von Sauerstoff im Polymerisationsgefäß kann durch Spülen mit inerten Gasen wie Stickstoff oder Argon erreicht werden.

Gemäß einer Ausführungsform der Erfindung kann es auch vorteilhaft sein, dem Reaktionsgemisch zur völligen Entfernung von gelöstem Sauerstoff - in dem Mengenbereich von 0,05 bis 1 Gew.-%, bezogen auf die Monomermischung - ein Reduktionsmittel wie Natriumsulfit, Natriumpyrosulfit, Natriumdithionit, Ascorbinsäure oder Mischungen der Reduktionsmittel, zuzusetzen.

Bevorzugt ist eine Polymerisation in Abwesenheit eines Initiators. Die radikalische Polymerisation wird also bevorzugt nicht als gestartete Polymerisation geführt. Wenn ein radikalbildender Initiator eingesetzt wird, dann nur in geringer Menge (z.B. J.W. Breitenbach et al.,"Die Makromolekulare Chemie", 175, 2597-2604 (1974):). Erfindungsgemäß bevorzugt werden also Polyacrylate, die durch eine spontane radikalische Polymerisation erhalten worden sind, als Sprengmittel für feste pharmazeutische Darreichungsformen verwendet.

Die Polymerisation erfolgt in wässrigem Medium. Gemäß einer bevorzugten Ausführungsform wird die Polymerisation als Fällungspolymerisation aus wässriger Lösung durchgeführt.

Die Konzentration der Monomere in der wässrigen Lösung kann 5 bis 90 Gew.-%, vorzugsweise 10 bis 80 Gew.-%, bezogen auf die gesamte Mischung, betragen. Gemäß einer bevorzugten Ausführungsform liegt die Monomerenkonzentration im Bereich von 10 bis kleiner 40 Gew.-%. Gemäß einer besonders bevorzugten Ausführungsform liegt die Monomerenkonzentration im Bereich von 10 bis 30 Gew.-%.

Die Polymerisate enthalten vorzugsweise zusätzlich einen Vernetzer. Als Vernetzer eignen sich Verbindungen, die mindestens zwei ethylenisch ungesättigte Doppelbindungen im Molekül enthalten. Besonders geeignet sind Methylenbisacrylamid, N,N'-Acryloylethylendiamin, Ethylenglykoldiacrylat, Ethylenglykoldimethacrylat, Tetraethylenglykolacrylat, Tetraethylenglykoldimethacrylat, Diethylenglykolacrylat, Diethylenglykolmethacrylat, Butandioldiacrylat, Hexandioldimethacrylat, Trimethylolpropantriacrylat, Dimethylmalonsäuredivinylester, Polyallylether von Saccharose, Pentaerythrittriallylether sowie Gemische der Vernetzer. Bevorzugt sind mindestens trifunktionelle Vernetzer. Besonders bevorzugt als Vernetzer sind Polyallylether von Saccharose und insbesondere Pentaerithrytoltriallylether.

Die Vernetzermenge kann 0,1 bis 15 Gew.-%, vorzugsweise 0,5 bis 10 Gew.-%, besonders bevorzugt 1 bis 5 Gew.-% bezogen auf die Monomermenge betragen.

Metallionen können die Popcornbildung verhindern. Es könnte deswegen von Vorteil sein, die Polymerisation in Gegenwart von Komplexbildnern durchzuführen. Beispiele für geeignete Komplexbildner sind das Natrium-Salz von Ethylendiamintetraessigsaure und Natriumpyrophosphat.

Die Polymerisationstemperatur kann in einem weiten Bereich variiert werden, z. B. von etwa 20 bis 200, vorzugsweise 50 bis 150°C.

Die Polymerisation erfolgt unter Durchleiten eines inerten Gases wie vorzugsweise Stickstoff. Der Reaktionsdruck stellt sich entsprechend ein.

Wenn Saat eingesetzt wird, liegt die mittlere Teilchengröße im Bereich von 0,5 bis 2 mm bestimmt durch dynamische Bildanalyse; mit einem Camsizer P4 Gerät der Firma Retsch).

Bevorzugt werden Popcorn-Polymerisate von Acrylsäure verwendet. Besonders bevorzugt sind Popcorn-Polymerisate der Acrylsäure mit Pentaerithrytoltriallylether als Vernetzer.

Die Popcorn-Polymerisate können im Anschluss an die Polymerisation auch teilneutralisiert werden. Dabei können je nach Anwendungsgebiet Neutralisationsgrade von 0 bis 80% eingestellt werden. Zur Teilneutralisation können wässrige Alkalilaugen wie Natronlauge oder Kalilauge (z.B. 1-25 Gew.-%-ig) oder wässrige Ammoniak Lösungen (z.B. 1-25 Gew.-%ig) verwendet werden.

Die sich bildenden Popcorn-Polymerpartikel können nach Beendigung der Polymerisation auf übliche Weise abgetrennt werde, beispielsweise durch Filtration oder Zentrifugation.

Anschließend können die Popcorn-Polymerpartikel noch mit Wasser gewaschen werden, um Restmonomere oder sonstige Verunreinigungen zu entfernen. Wässrige Polyacrylsäure Popcorn-Suspensionen enthalten < 1% Rest-Acrylsäure (bestimmt mit HPLC).

Zur Anwendung als Tablettensprengmittel werden die Popcorn-Polymerisate zerkleinert, vorzugsweise durch Mahlen. Dafür eignen sich übliche Mahlvorrichtungen zur Erzeugung von Pulvern.

Die durch Mahlung erhaltenen Polymerpulver können anschließend durch Siebung fraktioniert werden. Für die Anwendung als Tablettensprengmittel werden vorzugsweise die Siebfraktionen 100 bis 200 µm verwendet.

Gemäß einer bevorzugten Ausführungsform können Siebfraktionen mit Teilchengrößen von < 100 µm, 100-200 µm und 200-500 µm auch als Saatgut zur Initiierung der Popcorn-Polymerpartikelbildung eingesetzt werden. Solches Saatgut kann in Mengen von bis 0,1-10 Gew-.% bezogen auf die Monomermenge, eingesetzt werden.

Die Hilfsstoffe zur Tablettierung setzen sich vor allem zusammen aus Füllstoffen, Bindemitteln, Schmiermitteln und Sprengmitteln. Bei Verarbeitung sehr geringer Wirkstoffmengen (z.B. Alkaloide, Hormone, Vitamine usw.) werden Füllstoffe verwendet. Diese sorgen dafür, dass die Tablette die notwendige Größe bzw. notwendige Masse erhält. Eingesetzt werden Stärken wie z. B. Mais-, Kartoffel- und Weizenstärke, Lactose, mikrokristalline Cellulosen und für z. B. Lutschtabletten: Glucose, Mannitol, Sorbitol. Bindemittel (z. B. mikrokristalline Cellulose, Polyvinylpyrrolidon, Stärken usw.) sorgen darüber hinaus für den Zusammenhalt der Pulverpartikel in einem Granulat und beeinflussen die Festigkeit der Tabletten. Schmiermittel (z. B. Magnesiumstearat, Natriumstearylfumarat usw.) haben die Funktion, das Ausstoßen der Tablette aus der Matrize dadurch zu erleichtern, dass die Reibung zwischen Innenwand der Matrizenbohrung und Tablettenseitenfläche herabgesetzt wird. Zusätzlich wird die Reibung zwischen der Matrizenbohrung und dem Unter-stempel verringert, um ein Festfressen des Unterstempels zu verhindern. Die Zugabe von Sprengmitteln (z.B. Crospovidone, Croscarmellose-Na, Na-Stärkeglycolat usw.) verbessert das Verpressen zu haltbaren Tabletten (stärkere Partikelhaftung) und das spätere Zerfallen der Tabletten im Magen-Darm-Trakt.

Unter der Direkttablettierung ist das Verpressen von pulverförmigen Arzneistoff-Hilfsstoff-Mischungen ohne Vorbehandlung, gegenüber der Verpressung von hergestellten Granulaten, zu verstehen. Da diese Methode sich durch einen geringen Arbeitsaufwand auszeichnet, kam die Direkttablettierung in dieser Versuchsreihe zur Anwendung. Überraschenderweise wurde gefunden, dass vernetzte Poly(meth)acrylsäure-Produkte vom Typ eines Popcorn-Polymerisats mit einer Gelbildungszeit > 30 Sekunden eine bessere Sprengmittelleistung zeigen als vernetzte Polyacrylate mit einer Gelbildungszeit < 30 Sekunden wie sie aus dem Stand der Technik bekannt sind.

Die erfindungsgemäß verwendeten Popcorn-Polymerisate weisen auch eine deutlich bessere Sprengmittelleistung auf als herkömmliche kommerzielle Sprengmittel auf Basis von Stärke- oder Cellulosederivaten.

Vorteilhaft ist auch der im Vergleich zu Amberlite IRP 88 niedrigere pH-Wert bei Polymerisaten mit geringerem Neutralisationsgrad. Die erfindungsgemäß verwendeten Polyacrylat- Popcornpolymerisate zeigen eine gute Sprengwirkung, ohne dass hydrolyseempfindliche Wirkstoffe in ihrer Stabilität zu sehr beeinträchtigt würden.

Ganz besonders vorteilhaft ist aber die Sprengmittelwirkung der erfindungsgemäß verwendeten Polymerisate dann, wenn keine hydrolyseempfindlichen Wirkstoffe verarbeitet werden sollen. In diesem Falle kann man den Neutralisationsgrad deutlich erhöhen, was überraschenderweise eine signifikant bessere Sprengmittelwirkung nach sich zieht.

Die vorliegende Erfindung ist demnach insbesondere durch die folgenden Ausführungsformen charakterisiert, wobei jede Ausführungsform alle Merkmale der Ausführungsformen, auf die sie sich bezieht, enthält.

### Ausführungsform 1:

Verwendung von pulverförmigen, vernetzten, wasserunlöslichen, wenig quellenden Polyacrylaten als Sprengmittel für pharmazeutische feste Dosierungsformen.

### Ausführungsform 2:

Verwendung nach Ausführungsform 1, wobei es sich bei den Polyacrylaten um Popcorn-Polymerisate handelt.

Ausführungsform 3: Verwendung nach Ausführungsform 1 oder 2, wobei wenig quellend bedeutet, dass die prozentuale Änderung der Teilchengröße der Polymere in Wasser im Vergleich zur Änderung der Teilchengröße in Hexadecan bei kleiner 50 % liegt.

Ausführungsform 4: Verwendung nach einer der Ausführungsformen 1 bis 3, wobei die Polyacrylate aus Strukturelementen von Acrylsäure oder Methacrylsäure oder Gemischen davon bestehen.

Ausführungsform 5: Verwendung nach einem der Ausführungsformen1 bis 4, wobei die Polyacrylate bis zu 20 Gew.-% an Strukturelementen von Comonomeren enthalten.

Ausführungsform 6: Verwendung nach einem der Ausführungsformen 1 bis 5, wobei Strukturelemente von Comonomeren ausgewählt sind aus der Gruppe bestehend aus (Meth)acrylsäureestern wie Methylacrylat, Methylmethacrylat, Hydroxyethylmethacrylat, Hydroxyethylacrylat und Ethylacrylat, Butylacrylat, 2-Ethylhexylacrylat.

Ausführungsform 7: Verwendung nach einer der Ausführungsformen 1 bis 6, wobei pulverförmige Polyacrylate mit mittleren Teilchengrößen im Bereich von 100 bis 200 µm eingesetzt werden.

Ausführungsform 8: Verwendung nach einer der Ausführungsformen 1 bis 7, wobei die Polyacrylate zusätzlich Strukturelemente aus einem Vernetzer enthalten.

Ausführungsform 9: Verwendung nach einer der Ausführungsformen 1 bis 8, wobei der Vernetzer ausgewählt ist aus der Gruppe bestehend aus Methylenbisacrylamid, N,N'-Acryloylethylendiamin, Ethylenglykoldiacrylat, Ethylenglykoldimethacrylat, Tetraethylenglykolacrylat, Tetraethylenglykoldimethacrylat, Diethylenglykolacrylat, Diethylenglykolmethacrylat, Butandioldiacrylat, Hexandioldimethacrylat, Trimethylolpropantriacrylat, Dimethylmalonsäuredivinylester, Polyallylether von Saccharose, Pentaerythrittriallylether sowie Gemischen davon.

Ausführungsform 10: Verwendung nach einer der Ausführungsformen 1 bis 9, wobei der Vernetzer mindestens tri-funktionell ist.

Ausführungsform 11: Verwendung nach einer der Ausführungsformen 1 bis 10, wobei der Vernetzer Pentaerithrytoltriallylether ist.

Ausführungsform 12: Verwendung nach einer der Ausführungsformen 1 bis 11, wobei die Polyacrylate 0,1 bis 15 Gew.-%, bezogen auf die Menge an Acrylsäure oder Methacrylsäure, eines Vernetzers enthalten

Ausführungsform 13: Verwendung nach einer der Ausführungsformen 1 bis 12, wobei die Polyacrylate 0,5 bis 10 Gew.-%, bezogen auf die Menge an Acrylsäure oder Methacrylsäure, eines Vernetzers enthalten.

Ausführungsform 14: Verwendung nach einer der Ausführungsformen 1 bis 13, wobei die Polyacrylate 1 bis 5 Gew.-%, bezogen auf die Menge an Acrylsäure oder Methacrylsäure, eines Vernetzers enthalten.

Ausführungsform 15: Verwendung nach einer der Ausführungsformen 1 bis 14, wobei die Polyacrylate eine Gelbildungszeit von weniger als 30 Sekunden aufweisen.

Ausführungsform 16: Verwendung nach einer der Ausführungsformen 1 bis 15, wobei die Polyacrylate eine Gelbildungszeit von weniger als 30 Sekunden aufweisen und als Gelbildungszeit die Zeit bestimmt wird, nach der die durch das Rühren verursachte Wirbelröhre in einer Testflüssigkeit nicht mehr sichtbar ist, wobei als Testflüssigkeit eine 0,9 Gew.-%ige Natriumchloridlösung verwendet wird.

Ausführungsform 17: Pharmazeutische feste Dosierungsform gemäß der Verwendung nach einer der Ausführungsformen 1 bis 16, enthaltend als Sprengmittel pulverförmige wasserunlösliche, vernetzte Polyacrylate.

Ausführungsform 18: Pharmazeutische feste Dosierungsform nach Ausführungsform 17, enthaltend die als Sprengmittel wirkenden Polyacrylate in Mengen von 0.1 bis 50 Gew.-%, vorzugsweise 0.2 bis 20 Gew.-%, besonders bevorzugt 0.5 bis 5 Gew.-%, insbesondere 0.5 bis 2 Gew.-%, bezogen auf das Gesamtgewicht der Dosierungsform.

Ausführungsform 19: Verfahren zur Herstellung der gemäß einer der Ausführungsformen 1 bis 18 verwendeten Polyacrylate durch radikalische Polymerisation in wässrigem Medium.

Ausführungsform 20: Verfahren nach Ausführungsform 19, durch radikalische Polymerisation in wässrigem Medium, wobei die Polymerisation ohne Zugabe eines Radikalinitiators erfolgt.

Ausführungsform 21: Verfahren nach einer der Ausführungsformen 19 oder 20, wobei die Polymerisation im wässrigen Medium in Abwesenheit von Sauerstoff erfolgt.

Ausführungsform 22: Verfahren nach einer der Ausführungsformen 19 bis 21, wobei die Polymerisation im wässrigen Medium in Gegenwart eines Reduktionsmittels erfolgt.

Ausführungsform 23: Verfahren nach einer der Ausführungsformen 19 bis 22, wobei die Polymerisation im wässrigen Medium in Gegenwart eines Reduktionsmittels erfolgt und das Reduktionsmittel ausgewählt ist aus der Gruppe bestehend aus Natriumsulfit, Natriumpyrosulfit, Natriumdithionit, Ascorbinsäure oder Mischungen davon.

Ausführungsform 24: Verfahren nach einer der Ausführungsformen 19 bis 23, wobei die Polymerisation im wässrigen Medium in Gegenwart von 0.05 bis 1 Gew.-% eines Reduktionsmittels erfolgt.

Ausführungsform 25: Verfahren nach einer der Ausführungsformen 19 bis 24, wobei die Polymerisation in Gegenwart von Saatgut durchgeführt wird, wobei als Saatgut Partikelfraktionen der erfindungsgemäß verwendeten Polyacrylate eingesetzt werden.

Ausführungsform 26: Verfahren nach einer der Ausführungsformen 19 bis 25, wobei die Polymerisation in Gegenwart von Komplexbildnern durchgeführt wird.

Ausführungsform 27: Verfahren nach einer der Ausführungsformen 19 bis 26, wobei die Polymerisation in Gegenwart von Komplexbildnern wie dem Natrium-Salz von Ethylendiamintetraessigsäure oder Natriumpyrophosphat durchgeführt wird.

Ausführungsform 28: Verfahren nach einer der Ausführungsformen 19 bis 27, wobei die Polyacrylate teilneutralisiert werden

Ausführungsform 29: Verfahren nach einer der Ausführungsformen 19 bis 28, wobei die Polyacrylate mit wässrigen Alkalilaugen wie Natronlauge, Kalilauge oder wässrigen Ammoniaklösungen teilneutralisiert werden

Ausführungsform 30: Verfahren nach einer der Ausführungsformen 28 oder 29, wobei die Polyacrylate auf Neutralisationsgrade von bis zu 80 % eingestellt werden.

### Beispiele

Pentaerithrytoltriallylether (PETAE) ist kommerziell erhältlich von Perstorp GmbH. Angaben der Hersteller: Diallylethergehalt: 4.0-16.0%, Triallylethergehalt: 75.0-84.0% und Tetraallylethergehalt: 5.0-12.0%.

### VE-Wasser: vollentsalztes Wasser

### Herstellung von Popcorn Polymerisat A:

In einem 3 Liter-Glasreaktor, ausgerüstet mit einem Rührer, einem Rückflusskühler und Dosiereinrichtungen wurden 1086 g entionisiertes Wasser, 450 g Acrylsäure, 22,5 g Pentaerithrytoltriallylether (PETAE) und 2,25 g Natriumpyrophosphat vorgelegt und unter Rühren mit einer Drehzahl von 180 UpM auf 50°C erhitzt. Die Lösung wurde schon während der Aufheizperiode bis zum Abschluss der Polymerisation mit Stickstoff durchströmt, der mit Hilfe eines Rohres, das bis zum Boden der Rührapparatur reichte, in die Reaktionsmischung eingeführt wurde. Die Strömungsgeschwindigkeit betrug 20 L/Stunde. Nachdem die Temperatur der Reaktionsmischung 50°C erreicht hatte, wurden 1,04 g Natriumdithionit zugesetzt. Die Mischung wurde auf 50°C gehalten. Die ersten Popcorn-Polymerteilchen wurden 60 Minuten nach der Natriumdithionit- Zugabe gebildet, worauf eine Wärmeentwicklung auftrat, die weitere 70 Minuten dauerte. Währenddessen wurde die Mischung weiterhin gerührt. Die wässrige Suspension wurde anschließend noch eine weitere Stunde bei 50 °C gerührt und abfiltriert. Das Polymerisat wurde drei Mal mit 2000 ml Wasser gewaschen, um Verunreinigungen wie lösliches Polymer und restliche Monomere zu entfernen. Das Produkt wurde vor 12 Stunden bei 75 °C unter Vakuum getrocknet. Die Ausbeute an Popcornpolymerisat betrug 93%. Das Popcornpolymerisat zeigte eine sehr heterogene Teilchengrößen-Verteilung und enthält 5-10 cm große Partikel. Das Produkt wurde mit einem Thermomix der Fa. Vorwerk 4 Minuten bei maximaler Geschwindigkeit gemahlen und anschließend gesiebt. Die 100-200 µm Siebfraktion wurde als Sprengmittel getestet.

### Herstellung von Popcorn Polymerisat B:

Die Herstellung von Popcorn Polymerisat B erfolgte analog der Herstellung von Popcorn Polymerisat A, wobei 4,50 g der 100-200 µm-Siebfraktion von Polymerisat A als Saat in die Vorlage gegeben wurden. Die ersten Popcorn-Polymerteilchen waren schon nach 10 Minuten erkennbar. Die Ausbeute an Popcornpolymerisat betrug 91%. Die Verwendung von Saat führt zu einem deutlich feineren Produkt mit einer mittleren Teilchengröße im Bereich von 1 mm.

### Herstellung von Popcorn Polymerisat C:

Die Herstellung von Popcorn Polymerisat C erfolgte analog der Herstellung von Popcorn Polymerisat B, wobei die Herstellung ohne die Verwendung von 2,25 g Natriumpyrophosphat erfolgte. Die ersten Popcorn-Polymerteilchen waren nach 10 Minuten erkennbar. Die Ausbeute an Popcornpolymerisat betrug 88%.

### Herstellung von Popcorn Polymerisat D:

Die Herstellung von Popcorn Polymerisat D erfolgte analog der Herstellung von Popcorn Polymerisat A, wobei 2,25 g einer 200-500 µm-Siebfraktion von Polymerisat A als Saat in die Vorlage gegeben wurden. Die ersten Popcornpolymerteilchen waren nach 20 Minuten erkennbar. Die Ausbeute an Popcornpolymerisat betrug 94%.

### Herstellung von Popcorn Polymerisat E:

Die Herstellung von Popcorn Polymerisat E erfolgte analog der Herstellung von Popcorn Polymerisat A, wobei zunächst nur 90 g Acrylsäure vorgelegt wurden. Die restlichen 360 g Acrylsäure wurden nach der Bildung der ersten Popcornteilchen, welche 10 Minuten nach Natriumdithionit-Zugabe eintrat, innerhalb von zwei Stunden bei konstanter Dosiergeschwindigkeit zudosiert. Die Ausbeute an Popcornpolymerisat betrug 95%.

### Herstellung von Popcorn Polymerisat F:

Die Herstellung von Popcorn Polymerisat F erfolgte analog der Herstellung von Popcorn Polymerisat A, wobei 4,50 g der Siebfraktion < 100 µm von Polymerisat A als Saat in die Vorlage gegeben wurden. Die ersten Popcorn-Polymerteilchen waren nach 30 Minuten erkennbar. Die Ausbeute an Popcornpolymerisat betrug 92%.

### Herstellung von Popcorn Polymerisat G:

Die Herstellung von Popcorn Polymerisat G erfolgte analog der Herstellung von Popcorn Polymerisat F, wobei das Polymerisat teilneutralisiert wurde. Die Teilneutralisation erfolgte durch Zugabe von 300 g einer 25 Gew.- %igen wässrigen Natriumhydroxid-Lösung in die wässrige Suspension nach der Polymerisation, gefolgt von einer Stunde Rühren bei Raumtemperatur. Die ersten Popcorn Polymerteilchen waren nach 30 Minuten erkennbar.

Die Ausbeute an Popcornpolymerisat betrug 91%.

### Herstellung von Popcorn Polymerisat H:

Die Herstellung von Popcorn Polymerisat H erfolgte analog der Herstellung von Popcorn Polymerisat B, wobei 450 g Methacrylsäure anstelle von 450 g Acrylsäure eingesetzt wurden. Die ersten Popcorn Polymerteilchen waren nach 23 Stunden erkennbar. Die Reaktionsmischung wurde nachträglich 8 Stunden bei 80 °C gerührt. Die Filtration, Trocknung und Mahlung erfolgte analog 689. Die Ausbeute an Popcornpolymerisat betrug 62%.

### Herstellung von Popcorn Polymerisat I:

Die Herstellung von Popcorn Polymerisat I erfolgte analog der Herstellung von Popcorn Polymerisat B, wobei das Polymerisat teilneutralisiert wurde. Die Teilneutralisation erfolgte durch Zugabe von 420 g einer 25 Gew.-%igen wässrige Kaliumhydroxid-Lösung in die wässrige Suspension nach der Polymerisation, gefolgt von einer Stunde Rühren bei Raumtemperatur. Die ersten Popcorn-Polymerteilchen waren nach 30 Minuten erkennbar. Die Ausbeute an Popcornpolymerisat betrug 96%.

### Herstellung von Popcorn Polymerisat J:

Die Herstellung von Popcorn Polymerisat J erfolgte analog der Herstellung von Popcorn Polymerisat F, unter Verwendung der folgenden Mengen an Einsatzstoffen: 1050 g entionisiertes Wasser, 250 g Acrylsäure, 12,5 g PETAE, 1,25 g Natriumpyrophosphat, 2,50 g einer Siebfraktion von < 100 µm Fraktion von Polymerisat A als Saat und 0,58 g anstelle Natriumdithionit. Die ersten Popcorn-Polymerteilchen waren nach 20 Minuten erkennbar. Die Ausbeute an Popcornpolymerisat betrug 85%.

### Herstellung von Popcorn Polymerisat K:

Die Herstellung von Popcorn Polymerisat K erfolgte analog der Herstellung von Popcorn Polymerisat J, wobei als Saat 2,50 g der 100-200 µm-Siebfraktion von Polymerisat A verwendet wurde. Die ersten Popcorn Polymerteilchen waren nach 20 Minuten erkennbar.

Die Ausbeute an Popcornpolymerisat betrug 76%.

### Herstellung von Popcorn Polymerisat L:

Die Herstellung von Popcorn Polymerisat L erfolgte analog der Herstellung von Popcorn Polymerisat B unter Verwendung der folgenden Mengen an Einsatzstoffen: 1300 g entionisiertes Wasser, 300 g Acrylsäure, 15,0 g PETAE, 1,50 g Natriumpyrophosphat, 3,00 g der 100-200 µm-Siebfraktion von Polymerisat A als Saat und 0,69 g Natriumdithionit. Weiterhin wurde das Polymerisat teilneutralisiert.

Die Teilneutralisation erfolgte durch Zugabe von 200 g einer 25 Gew.- %igen wässrigen Kaliumhydroxid-Lösung in die wässrige Suspension nach der Polymerisation, gefolgt von eine Stunde Rühren bei Raumtemperatur. Die ersten Popcorn-Polymerteilchen waren nach 35 Minuten erkennbar. Die Ausbeute an Popcornpolymerisat betrug 85%.

### Herstellung von Popcorn Polymerisat M:

Die Herstellung von Popcorn Polymerisat M erfolgte analog der Herstellung von Popcorn Polymerisat J, wobei als Saat 2,50 g der Siebfraktion < 100 µm von Polymerisat A verwendet wurde, und dass das Polymerisat teilneutralisiert wurde. Die Teilneutralisation erfolgte durch Zugabe von 167 g einer 25 Gew.-%igen wässrigen Natriumhydroxid-Lösung in die wässerige Suspension nach der Polymerisation, gefolgt von einer Stunde Rühren bei Raumtemperatur. Die ersten Popcorn-Polymerteilchen waren nach 30 Minuten erkennbar. Die Ausbeute an Popcornpolymerisat betrug 90%.

Zum Vergleich: Herstellung von Gel Polymerisat V1:
In einem 2 Liter-Glasreaktor, ausgerüstet mit einem Rührer und einem Rückflusskühler wurden bei Raumtemperatur und unter Stickstoffatmosphäre 605 g entionisiertes Wasser, 400 g Acrylsäure, 20 g PETAE und 5 g einer 5 Gew-%igen wässerigen WasserstoffperoxidLösung bei Raumtemperatur vorgelegt. Die Mischung wurde bei 180 UpM gerührt und auf 30 °C erwärmt. Eine 25 Gew.-%ige wässerige Natriumhydroxid-Lösung (266,6 g) wurde innerhalb 20 Minuten zudosiert. Anschließend wurde 28,2 g von einer wässrigen L(+)-Ascorbinsäure-Lösung zugegeben (0,2 g L(+)-Ascorbinsäure in 28 g Wasser). Innerhalb weniger Sekunden waren eine sehr starke Wärmeentwicklung und Viskositätsanstieg zu beobachten. Die Rührgeschwindigkeit wurde auf 20 UpM reduziert. Die Viskosität stieg weiter an und es bildete sich ein homogenes klares Gel. Das Gel wurde zwei Stunden gerührt und anschließend aus dem Reaktor entfernt, zerkleinert und gefriertrocknet. Die Ausbeute an Gel-Polymerisat betrug 99 %. Das Produkt wurde mit einem Thermomix der Fa. Vorwerk 4 Minuten bei maximaler Geschwindigkeit gemahlen und anschließend gesiebt. Die 100-200 µm Siebfraktion wurde als Sprengmittel getestet (Tabelle 3).

### Herstellung von Placebo-Tabletten

Alle der im Folgenden angegeben Zerfallszeiten wurden an Placebo-Tabletten getestet, die wie folgt erhalten wurden:
Tablettenformulierung (Diekttablettierung):
467,50 mg Ludipress LCE (96,5 % Lactosemonohydrat, 3,5 % Polyvinylpyrrolidon (Kollidon^{®} 30)), 30 mg Sprengmittel (Popcorn und Gel-Polymerisate, Amberlite IRP 88, Ac-Di-Sol, Primojel, Kollidon CL) und 2,50 mg Magnesiumstearat. Alle Einsatzstoffe wurden über 0,8 mm Maschenweite gesiebt und 10 Minuten in einem Turbula Mischer gemischt. Tabletten wurden bei 18 kN auf einer Exzenterpresse Korsch XP1, bei einigen dieser Formulierungen bei 6 und 12 kN, gepresst. Zerfallszeiten wurden mit einem Erweka ZT 74 (Zerfallstester) in 0,08N HCl (pH 1,1), Phosphatpuffer (pH 6,8) und VE- Wasser bestimmt.

Die Messung der Zerfallszeit erfolgte mit dem Erweka ZT 74 gemäß der im Folgenden beschriebenen Methode: Ein 1000 ml Becherglas (niedrige Form) mit 800 ml des gewünschten Prüfmediums (z.B. 0,08 N HCl, Phosphatpuffer, VE- Wasser) füllen, im Wasserbad positionieren und auf 36-38°C temperieren. In jedes der nummerierten Röhrchen des Messkorbes wird eine Tablette oder Kapsel (sechsfach Bestimmung je Formulierung) und darauf die dazugehörige Disk gelegt. Dieser Messkorb wird über das gefüllte Becherglas in die Halterung gehängt und die Messung gestartet. Der Korb wird so lange hoch und runterfahren, bis in allen Röhrchen die Restdicke der Tablette nur noch max. 0,2 mm beträgt. Der Sensor liefert je nach Abstand des im Disk eingebauten Magneten ein Signal. Ist die Probe zerfallen, so ist der kleinstmögliche Abstand erreicht und die Zeit wird aufgezeichnet. Das Ende der Prüfung ist erreicht und die Zerfallszeit bestimmt, wenn alle Prüflinge zerfallen sind. Der Messkorb schaltet sich dann automatisch ab.

### Bestimmung der Gelbildungszeit

Die Bestimmung der Gelbildungszeit erfolgte wie in der EP-A 1035196, [0028], beschrieben. Die genaue Methode ist weiter oben in der allgemeinen Beschreibung ausgeführt.

### Bestimmung der Partikelgrößenverteilung (TGV) in Hexadecan und Wasser

Die Messungen wurden mit Mastersizer 3000 (Static Light Scattering/Fraunhofer Diffraction) nach ISO 13320:2009 durchgeführt.

### Methode 1: wässrig

Die Messeinheit für wässrige Proben ist die Hydroeinheit MV:
Für die Messung in der Hydroeinheit wurde nach dem Initialisierungsschritt und der Hintergrundmessung, die Probe (Mikrospatel) direkt in die Hydroeinheit gegeben, bis die optimale Laserabschattung von 2-15% erreicht war. Nach der Zugabe der Probe wurde ca. 30 Sekunden gewartet, bis die Probe sich gut dispergiert hat und dann mit der Messung gestartet. Die Parameter für Analyse und Auswertung in der Hydroeinheit MV:
Rührgeschwindigkeit: 1500 Upm
Ultraschall: ohne
Auswertemodell: Fraunhofer
Analyse Modell: Universal

| Probe | Medium Wasser Laserabschattung (%) |
|---|---|
| Gel-Polymerisat V1 | 6,49 |
| Polymerisat M | 9,33 |

### Methode 2: in Hexadecan

Die Messeinheit für Hexadecan ist die Küvetteneinheit SV:
Für die Messung in der Küvetteneinheit SV wurde nach der Initialisierung und Hintergrundmessung, die Probe (1 Löffelspatel Probe in ein 10 ml Schraubdeckelglas mit Hexadecan homogenisieren) tropfenweise zugegeben, bis die optimale Laserabschattung von 2-15% erreicht war. Nach Einbringen der Küvettenmeßeinheit in das Gerät, wurde die Messung gestartet. Die Parameter für Analyse und Auswertung in der Küvetteneinheit SV:
Rührgeschwindigkeit: 1500 Upm
Ultraschall: ohne
Auswertemodell: Fraunhofer
Analyse Modell: Universal

| Probe | Hexadecan Laserabschattung (%) |
|---|---|
| Gel-Polymerisat V1 | 6,78 |
| Polymerisat M | 12,50 |

Die Ergebnisse sind in den nachstehenden Tabellen aufgelistet.

**Tabelle 1: Vergleich von Zerfallszeiten der Tablettenformulierung von kommerziellen Sprengmitteln mit erfindungsgemäßem Polymerisat M**

| Sprengmittel | Zerfallszeit pH 1,1 [min:s] | Zerfallszeit pH 6,8 [min:s] | Zerfallszeit VE-Wasser [min:s] |
|---|---|---|---|
| Ac-Di-Sol (Croscarmellose-Na) | 2:56 | 3:50 | 2:39 |
| Primojel (Na-Stärkeglycolat) | 2:45 | 3:38 | 3:32 |
| Kollidon^{®} CL (Crospovidon) | 1:15 | 1:54 | 1:12 |
| Polymerisat M | 2:09 | 2:35 | 2:12 |

Das Polyacrylsäure Popcorn-Polymerisat M zeigte eine bessere Leistung als die momentan auf dem Markt verfügbaren kostengünstigen Sprengmittel-Produkte wie Ac-Di-Sol und Primojel.

**Tabelle 2: Gelbildungszeit, TGV in Hexadecan/Wasser und Zerfallszeit: Popcorn vs Gel Polymerisation**

| Sprengmittel | Gelbildungszeit* [min:s] | Teilchengroß e in Hexadecan [µm] | Teilchengroß e in Wasser [µm] | Teilchengroß e Änderung C₁₆H₃₄ → H₂O | Zerfallzei t pH 1,1 [min:s] |
|---|---|---|---|---|---|
| Gel-Polymerisat V1 | < 0:10 | 110 | 475 | + 332 % | 3:18 |
| Polymerisat M | >10:00 | 124 | 165 | + 33 % | 2:09 |

| | | | | | |
|---|---|---|---|---|---|
| *Beschrieben in EP1035196 B1 (Seite 5, [0028]) | | | | | |

Die Polymerisate wurden mit identischen Mengen an Monomer und Vernetzer hergestellt, unterscheiden sich jedoch deutlich. Die Ergebnisse in Tabelle 2 zeigen, dass das Gelpolymerisat in Wasser schnell und stark quillt. Das Popcorn-Polymerisat quillt nur wenig und sehr langsam, zeigt aber eine bessere Sprengmittelwirkung.

Weiterhin wurde die Sprengmittelwirkung von erfindungsgemäßen Polymerisaten mit der Sprengmittelwirkung des häufig verwendeten kommerziellen Amberlite^{™} IRP 88 verglichen. Die Ergebnisse sind in Tabelle 3 aufgelistet.

**Tabelle 3: Neutralisation, pH und Zerfallszeit: Popcorn vs Amberlite**

| Sprengmittel | Neutralisation [mol% Carboxylsäure Gruppen] | pH (1%ig in Wasser) | Zerfallszeit pH 1,1 [min:s] | Zerfallszeit pH 6,8 [min:s] | Zerfallsz eit VE-Wasser [min:s] |
|---|---|---|---|---|---|
| Amberlite IRP 88 | 60* | 9.7 | 2:25 | 2:24 | 2:42 |
| Polymerisat K | 0 | 3.4 | 1:54 | 2:00 | 1:40 |
| Polymerisat M | 30 | 7.7 | 2:09 | 2:35 | 2:12 |
| Polymerisat L | 50 | 9.0 | 1:14 | 1:01 | 0:48 |

| | | | | | |
|---|---|---|---|---|---|
| *) berechnet aus Wassergehaltsbestimmung und Elementaranalyse (Kalium und Sauerstoff) | | | | | |

**Tabelle 4: Bestimmung der Zerfallszeiten der Tablettenformulierungen bei unterschiedlichen pH-Werten**

| Polymerisat | Zerfallszeit pH 1,1 [min:s] | Zerfallszeit pH 6,8 [min:s] | Zerfallszeit VE-Wasser [min:s] |
|---|---|---|---|
| A | 1:34 | 1:32 | 1:12 |
| B | 1:18 | 1:42 | 1:13 |
| C | 1:28 | 1:42 | 1:21 |
| D | 2:33 | 2:06 | 1:28 |
| E | 2:07 | 2:12 | 1:52 |
| F | 1:30 | 1:32 | 1:13 |
| G | 1:54 | 2:11 | 1:56 |
| H | 3:44 | 4:30 | 3:55 |
| I | 2:29 | 1:45 | 1:52 |
| J | 2:00 | 2:09 | 1:41 |
| K | 1:54 | 2:00 | 1:40 |
| L | 1:14 | 1:01 | 0:48 |
| M | 2:09 | 2:35 | 2:12 |

## Patentansprüche

1. Verwendung von pulverförmigen, vernetzten, wasserunlöslichen, wenig quellendenPolyacrylaten als Sprengmittel für pharmazeutische feste Dosierungsformen, wobei wenig quellend erfindungsgemäß bedeutet, dass die prozentuale Änderung der Teilchengröße der Polyacrylate in Wasser im Vergleich zur Änderung der Teilchengröße in Hexadecan bei kleiner 50% liegt, es sich bei den Polyacrylaten um Popcorn-Polymerisate handelt und die Polyacrylate aus Strukturelementen von Acrylsäure oder Methacrylsäure oder Gemischen davon bestehen.

2. Verwendung nach Anspruch 1, wobei die Polyacrylate bis zu 20 Gew.-% an Strukturelementen von Comonomeren enthalten und die Strukturelemente von Comonomeren ausgewählt sind aus der Gruppe bestehend aus (Meth)acrylsäureestern wie Methylacrylat, Methylmethacrylat, Hydroxyethylmethacrylat, Hydroxyethylacrylat und Ethylacrylat, Butylacrylat, 2-Ethylhexylacrylat.

3. Verwendung nach einem der Ansprüche 1 oder 2, wobei pulverförmige Polyacrylate mit mittleren Teilchengrößen im Bereich von 100 bis 200µm eingesetzt werden.

4. Verwendung nach einem der Ansprüche 1 bis 3, wobei die Polyacrylate zusätzlich Strukturelemente aus einem Vernetzer enthalten.

5. Verwendung nach einem der Ansprüche 1 bis 4, wobei der Vernetzer mindestens tri-funktionell ist.

6. Verwendung nach einem der Ansprüche 1 bis 5, wobei der Vernetzer Pentaerithrytoltriallylether ist.

7. Verwendung nach einem der Ansprüche 1 bis 6, wobei die Polyacrylate 0,1 bis 15 Gew.-%, vorzugsweise 0,5 bis 10 Gew.-%, besonders bevorzugt 1 bis 5 Gew.-% bezogen auf die Menge an Acrylsäure oder Methacrylsäure, eines trifunkionellen Vernetzers enthalten

8. Pharmazeutische feste Dosierungsform gemäß der Verwendung nach einem der Ansprüche 1 bis 7, enthallend als Sprengmittel pulverförmige wasserunlösliche, vernetzte Polyacrylate.

## Claims

1. Use of powder-form, crosslinked, water-insoluble, low-swelling polyacrylates as disintegrants for solid pharmaceutical dosage forms, where "low-swelling" in the context of the invention means that the percent change in the polyacrylate particle size in water compared with the change in particle size in hexadecane is less than 50%, the polyacrylates are popcorn polymers, and the polyacrylates consist of structural elements of acrylic acid or methacrylic acid or mixtures thereof.

2. Use according to Claim 1, wherein the polyacrylates contain up to 20% by weight of comonomer structural elements and the comonomer structural elements are selected from the group consisting of (meth)acrylic esters such as methyl acrylate, methyl methacrylate, hydroxyethyl methacrylate, hydroxyethyl acrylate and ethyl acrylate, butyl acrylate, and 2-ethylhexyl acrylate.

3. Use according to either of Claims 1 or 2, wherein powder-form polyacrylates having an average particle size within the range from 100 to 200 µm are used.

4. Use according to any of Claims 1 to 3, wherein the polyacrylates additionally comprise structural elements from a crosslinker.

5. Use according to any of Claims 1 to 4, wherein the crosslinker is at least trifunctional.

6. Use according to any of Claims 1 to 5, wherein the crosslinker is pentaerythritol triallyl ether.

7. Use according to any of Claims 1 to 6, wherein the polyacrylates contain 0.1% to 15% by weight, preferably 0.5% to 10% by weight, more preferably 1% to 5% by weight, based on the amount of acrylic acid or methacrylic acid, of a trifunctional crosslinker.

8. Solid pharmaceutical dosage form in accordance with the use according to any of Claims 1 to 7 that comprises, as a disintegrant, powder-form water-insoluble crosslinked polyacrylates.

## Revendications

1. Utilisation de polyacrylates sous forme de poudre, réticulés, insolubles dans l'eau, peu gonflants, comme agent de désintégration pour des formes posologiques pharmaceutiques solides, peu gonflant signifiant selon l'invention que la modification, en pour cent, de la grosseur de particule des polyacrylates dans l'eau par rapport à la modification de la grosseur de particule dans l'hexadécane est inférieure à 50%, les polyacrylates étant des polymères de type popcorn et les polyacrylates étant constitués par des éléments structuraux d'acide acrylique ou d'acide méthacrylique ou leurs mélanges.

2. Utilisation selon la revendication 1, les polyacrylates contenant jusqu'à 20% en poids d'éléments structuraux de comonomères et les éléments structuraux de comonomères étant choisis dans le groupe constitué par les esters de l'acide (méth)acrylique tels que l'acrylate de méthyle, le méthacrylate de méthyle, le méthacrylate d'hydroxyéthyle, l'acrylate d'hydroxyéthyle et l'acrylate d'éthyle, l'acrylate de butyle, l'acrylate de 2-éthylhexyle.

3. Utilisation selon l'une des revendications 1 ou 2, des polyacrylates sous forme de poudre présentant des grosseurs moyennes de particule dans la plage de 100 à 200 um étant utilisés.

4. Utilisation selon l'une des revendications 1 à 3, les polyacrylates contenant en plus des éléments structuraux d'un réticulant.

5. Utilisation selon l'une des revendications 1 bis 4, le réticulant étant au moins trifonctionnel.

6. Utilisation selon l'une des revendications 1 à 5, le réticulant étant le pentaérithrytoltriallyléther.

7. Utilisation selon l'une des revendications 1 à 6, les polyacrylates contenant 0,1 à 15% en poids, de préférence 0,5 à 10% en poids, de manière particulièrement préférée 1 à 5% en poids, par rapport à la quantité d'acide acrylique ou d'acide méthacrylique, d'un réticulant trifonctionnel.

8. Forme posologique pharmaceutique solide selon l'utilisation selon l'une des revendications 1 à 7, contenant comme agent de désintégration des polyacrylates sous forme de poudre, insolubles dans l'eau, réticulés.
